# EUROPEAN PATENT APPLICATION

(11) **EP 2 269 629 A2**
(43) Date of publication of application: **05.01.2011**
(21) Application number: 10010087.4
(22) Date of filing: 11.05.2001
(51) Int. Cl.: A61K 38/20, A61K 38/19, A61K 38/18, C12N 5/0784

(54) **Compositions and methods for producing antigen-presenting cells**

(30) Priority: 11.05.2000 US 203571 P
(62) Divisional of application: 07016833.1
(71) Applicant: Baylor Research Institute, Dallas, TX 75204 (US)
(72) Inventor: Bancheraeu, Jacques F., Dallas, TX 75230 (US); Mohamadzadeh, Mansour, Plano, TX 75093 (US); Palucka, Anna Karolina, Dallas, TX 75204 (US)
(74) Representative: UEXKÜLL & STOLBERG

(57) **Abstract**

The present invention relates to compositions and methods for producing antigen-presenting cells, in vitro, ex vivo or in vivo. This invention relates more particularly to methods and compositions for producing dendritic cells using interleukin-15, preferably in combination with a growth factor such as GM-CSF. This invention is particularly suited for producing immature dendritic cells and activated cells from precursors, in vitro, ex vivo or in vivo. The invention also relates to compositions for implementing these methods, as well as compositions comprising antigen-presenting cells and uses thereof. Dendritic cells or membrane vesicles derived thereforom have utility in many applications, including diagnostic, therapy, vaccination, research, screening and gene delivery.

## Description

### CROSS-REFERENCE TO RELATED APPLICATION

This application claims the benefit of U.S. Provisional Patent Application No. 60/203,571, filed May 11, 2000.

### TECHNICAL FIELD OF INVENTION

The present invention relates to compositions and methods for producing antigen-presenting cells, in vitro, ex vivo or in vivo. This invention relates more particularly to methods and compositions for producing antigen-presenting cells using interleukin-15 (IL-15), preferably in combination with a growth factor such as Granulocyte-Macrophage Colony Stimulating Factor (GM-CSF). This invention is particularly suited for producing dendritic cells from precursors in vitro, ex vivo or in vivo. The invention also relates to compositions for implementing these methods, as well as compositions comprising antigen-presenting cells and uses thereof.

### BACKGROUND OF THE INVENTION

The immune system comprises various cell populations, including antigen-presenting cells which are involved in antigen presentation to effector or regulatory cells, such as macrophages, B-lymphocytes and dendritic cells. Dendritic cells are potent antigen-presenting cells, involved in innate immunity, and capable of inducing antigen-specific cytotoxic T lymphocyte (CTL) responses. For this reason, dendritic cells are being proposed for use in immunotherapy of various pathologies, including tumors or immune diseases, through production of said cells in vitro, sensitization to an antigen, and re-infusion to a patient Also, membrane vesicles derived from dendritic cells have been discovered and isolated, which exhibit very powerful immunogenic activity (International Application No. WO99/03499). Current dendritic cell production methods essentially rely on the differentiation from precursor cells (essentially peripheral blood monocytes) by culture in the presence a combination of a cytokine and a growth factor, more specifically interleukin-4 or interleukin-13 in combination with Granulocyte-Macrophage Colony Stimulating Factor (GM-CSF). There is a need in the art to develop alternative production methods, which produce functional dendritic cells or functional antigen-presenting cells for use in any therapeutic, diagnostic, prophylactic or research area.

### SUMMARY OF INVENTION

In one aspect, the present invention is a method for producing dendritic cells from dendritic cell precursors in vitro or ex vivo comprising contacting dendritic cell precursors with a composition comprising interleukin-15 and at least one growth factor. In one embodiment, the resulting dendritic cells are immature. These immature dendritic cells can be sensitized to an antigen. These immature dendritic cells can be administered to a patient for modulating an immune response in the patient. The invention also includes a composition comprising immature dendritic cells produced according to this method.

In another aspect, the invention is a method for producing activated dendritic cells from dendritic cell precursors in vitro or ex vivo comprising contacting dendritic cell precursors with a composition comprising interleukin-15 and at least one growth factor to form activated dendritic cells. The activated dendritic cells can be sensitized to an antigen. These activated dendritic cells can be administered to a patient for modulating an immune response in the patient The invention also includes a composition comprising activated dendritic cells produced according to this method.

In another aspect, the invention is a method for producing mature dendritic cells from dendritic cell precursors in vitro or ex vivo comprising contacting dendritic cell precursors with a composition comprising interleukin-15 and at least one growth factor to form immature dendritic cells and culturing the immature dendritic cells in the presence of an maturation agent and under conditions to promote maturation of the immature dendritic cells to form mature dendritic cells. In one embodiment, the immature dendritic cells are sensitized to an antigen either prior to, during or after maturation. Exemplary maturation agents for in vitro use include lipopolysaccharide, CD40L, and poly(I):(C). These mature dendritic cells can be administered to a patient for modulating an immune response in the patient. The invention also includes a composition comprising mature dendritic cells produced according to this method.

In another aspect, the invention is a method for producing isolated membrane vesicles comprising contacting dendritic cell precursors with a composition comprising interleukin-15 and at least one growth factor to form immature dendritic cells, culturing the immature dendritic cells in the presence of at least one stimulation factor to promote the release of membrane vesicles; and isolating the membrane vesicles. In one embodiment, the immature dendritic cells are sensitized to an antigen prior to or after membrane vesicle release. Exemplary stimulation factors include a suitable cytokine, irradiation, and low pH. These membrane vesicles can be administered to a patient for modulating an immune response in the patient. The invention also includes a composition comprising membrane vesicles produced according to this method.

In another aspect, the invention is a method for producing immature dendritic cells from dendritic cell precursors in a patient comprising concurrent or sequential administration of interleukin-15 and at least one growth factor to the patient.

In another aspect, the invention is a method for producing activated dendritic cells from dendritic cell precursors in a patient comprising concurrent or sequential administration of interleukin-15 and at least one growth factor to the patient.

In another aspect, the invention is a method for producing mature dendritic cells from dendritic cell precursors in a patient comprising concurrent or sequential administration of interleukin-15, at least one growth factor, and at least one maturation factor to the patient.

In another aspect, the invention is a method for modulating an immune response in a patient via dendritic cells comprising concurrent or sequential administration of interleukin-15 and at least one growth factor. This method can also include administering at least one maturation factor to the patient

In another aspect, the invention is a method for inducing or stimulating dendritic cell differentiation from precursors thereof comprising contacting the precursors with interleukin-15 and at least one growth factor.

In another aspect, the invention is a method for producing antigen-specific cytotoxic T lymphocytes in vitro or ex vivo comprising providing antigen-sensitized dendritic cells prepared by contacting precursors thereof with interleukin-15 and at least one growth factor and subsequent sensitizing of the dendritic cells to an antigen, and contacting the antigen-sensitized dendritic cells with a population of cells comprising T lymphocytes to form activated antigen-specific cytotoxic T lymphocytes. These antigen-specific cytotoxic T lymphocytes can be administered to a patient for modulating an immune response in the patient. The invention also includes a composition comprising antigen-specific cytotoxic T lymphocytes produced according to this method.

In another aspect, the invention is a method for producing antigen-specific CD4+ T lymphocytes in vitro or ex vivo comprising providing antigen-sensitized dendritic cells prepared by contacting precursors thereof with interleukin-15 and a growth factor and subsequent sensitizing of the dendritic cells to an antigen and contacting the antigen-sensitized dendritic cells with a population of cells comprising T lymphocytes to form activated antigen-specific CD4+ T lymphocytes. These antigen-specific CD4+ T lymphocytes can be administered to a patient for modulating an immune response in the patient. The invention also includes a composition comprising antigen-specific CD4+ T lymphocytes produced according to this method.

In another aspect, the invention is a method for producing dendritic cell precursors comprising contacting monocytes in vitro or ex vivo with interleukin-15 to form dendritic cell precursors. The invention also includes a composition comprising dendritic cell precursors produced according to this method.

The interleukin-15 useful in the methods and compositions of the present invention is preferably selected from the group consisting of isolated interleukin-15, a recombinant interleukin-15 polypeptide, a nucleic acid, either wild type or engineered, encoding interleukin-15 in a suitable expression vector, and a cell population expressing a nucleic acid encoding interleukin-15. The growth factor useful in the methods and compositions of the present invention is preferably selected from the group consisting of isolated growth factor, a recombinant growth factor polypeptide, a nucleic acid encoding a growth factor in a suitable expression vector, and a cell population expressing a nucleic acid encoding a growth factor. More preferably, the growth factor is granulocyte-macrophage colony stimulating factor. The maturation factor useful in the present invention is preferably selected from the group consisting of lipopolysaccharide, CD40L, and poly(I):(C).

### BRIEF DESCRIPTION OF THE DRAWINGS

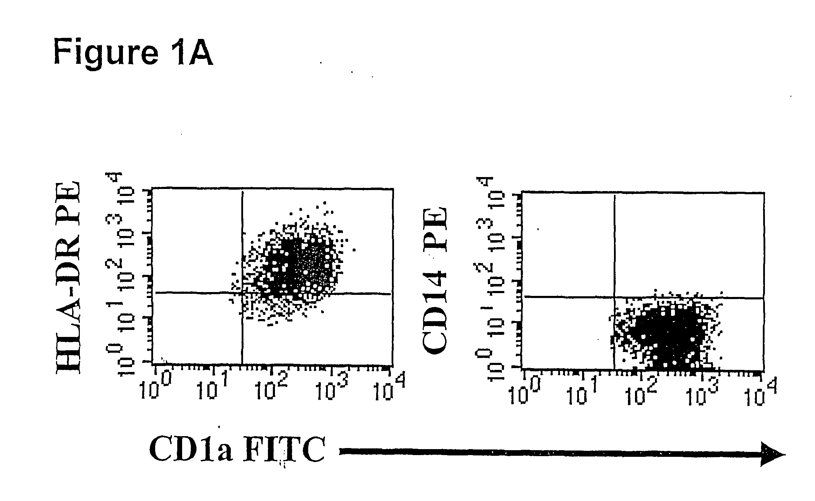

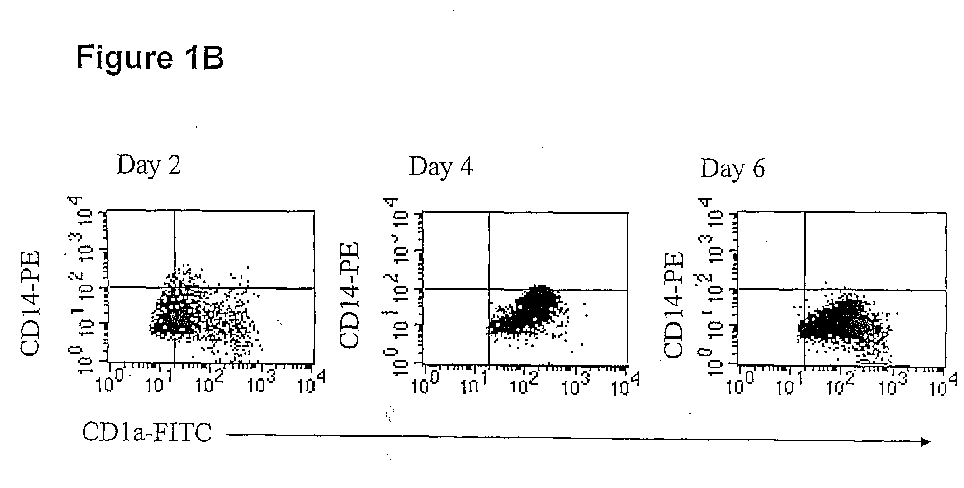

Fig. 1A and 1B depict the development of DCs in the presence of GM-CSF and IL-15 (GM-CSF+IL-15). Enriched CD14⁺ cells were cultured in the presence of GM-CSF and IL-15 over six days. Fig. 1A depicts non-adherent cells harvested and analyzed for CD1a, CD14, and HLA-DR by FACS Calibur (Becton Dickinson, San Jose, CA, US). Fig. 1B depicts cultured monocytes in GM-CSF and IL-15 analyzed in a time kinetic manner by FACS Calibur.

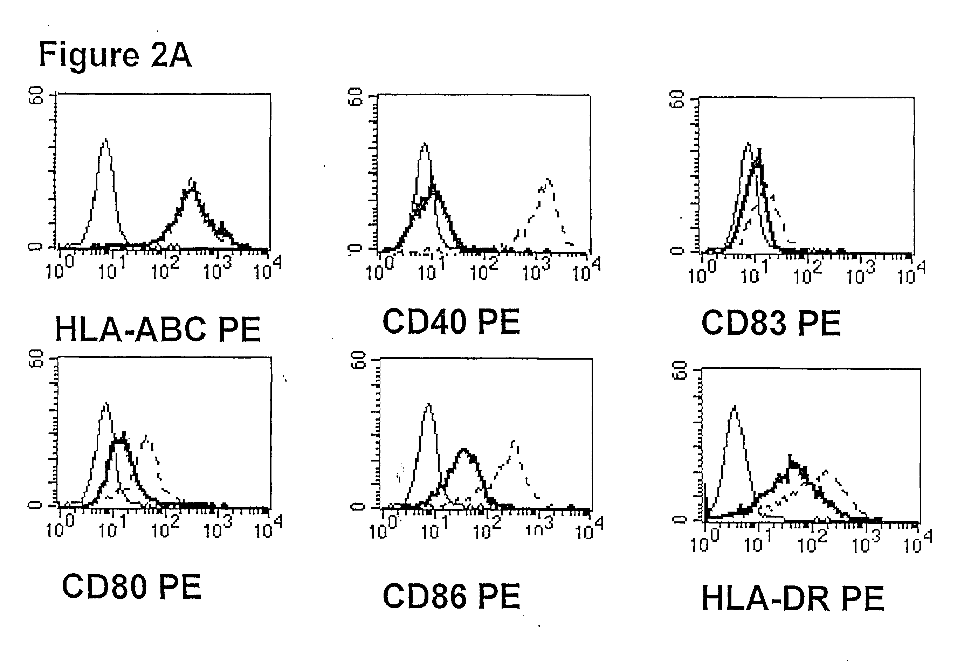

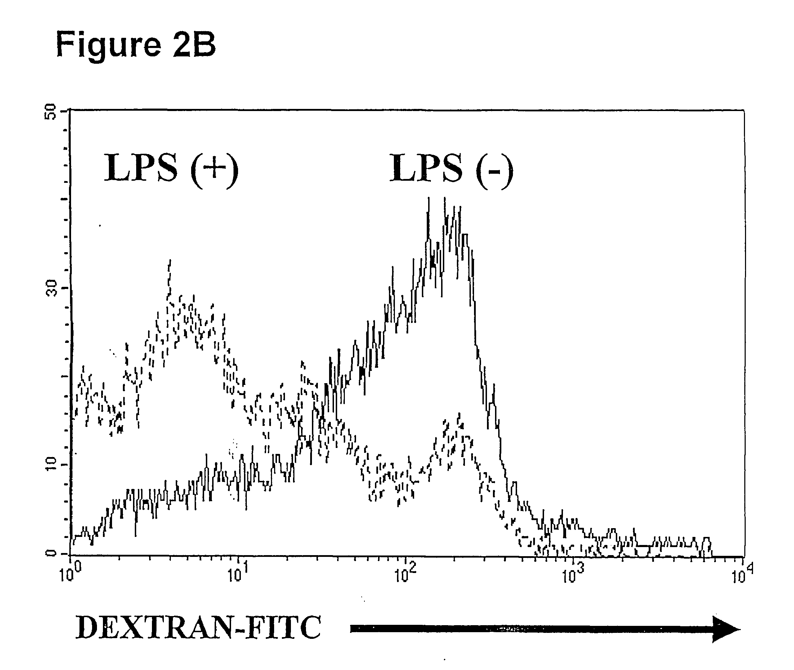

Fig. 2A and 2B depict maturation of GM-CSF+IL-15-derived DCs with lipopolysaccharide (LPS). Fig. 2A depicts the results obtained on Day 5 when a part of GM-CSF+IL-15-derived DCs were activated with LPS (100 ng/ml) and analyzed for HLA-ABC, CD40, CD80, CD83, and CD86. Fig. 2B depicts the results of FITC-Dextran up-take studies. Activated (dotted line) or non activated DCs (solid line) were incubated with 100 µg/ml FITC-Dextran for 30 min at 37°C. Control DCs were incubated on ice for 30 min as well. Afterwards, DCs were washed three times with PBS and analyzed for FITC-Dextran up-take by FACS Calibur.

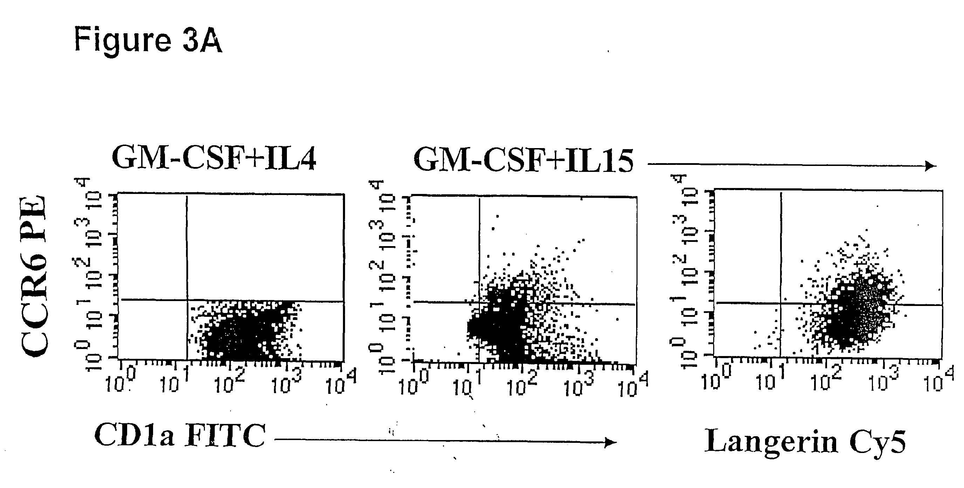

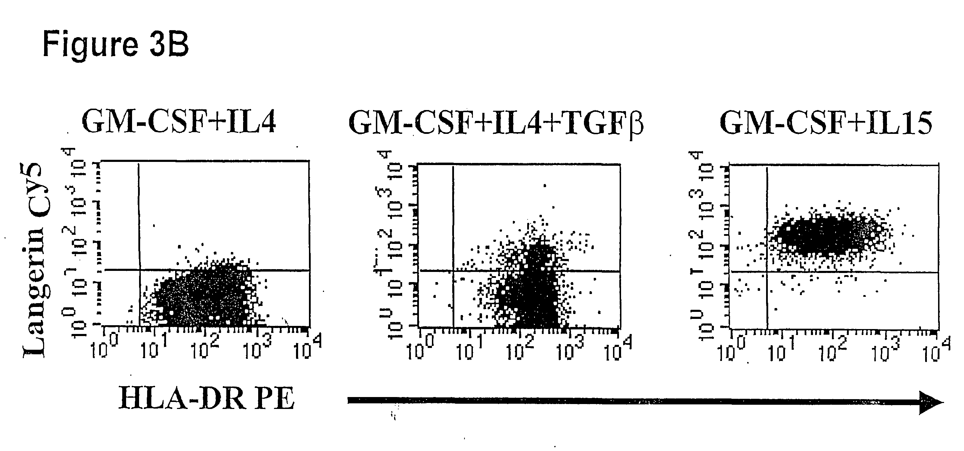

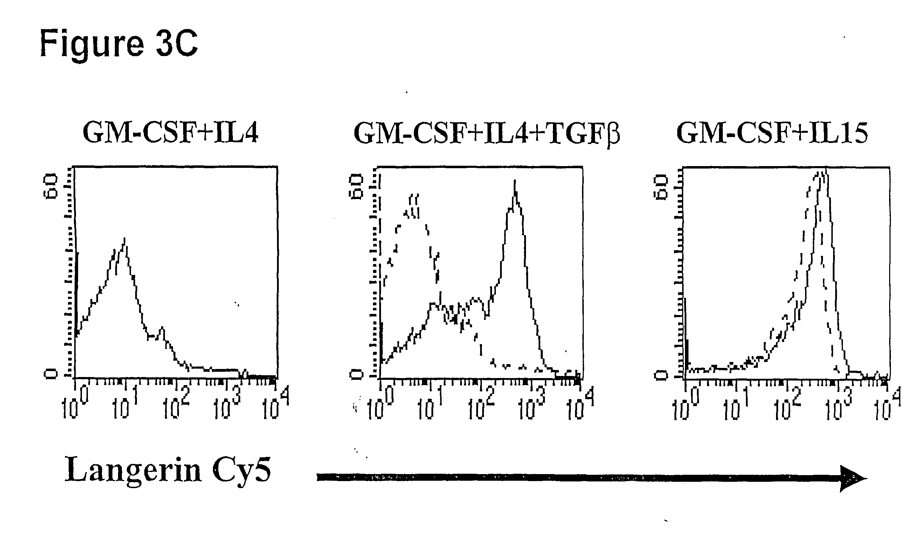

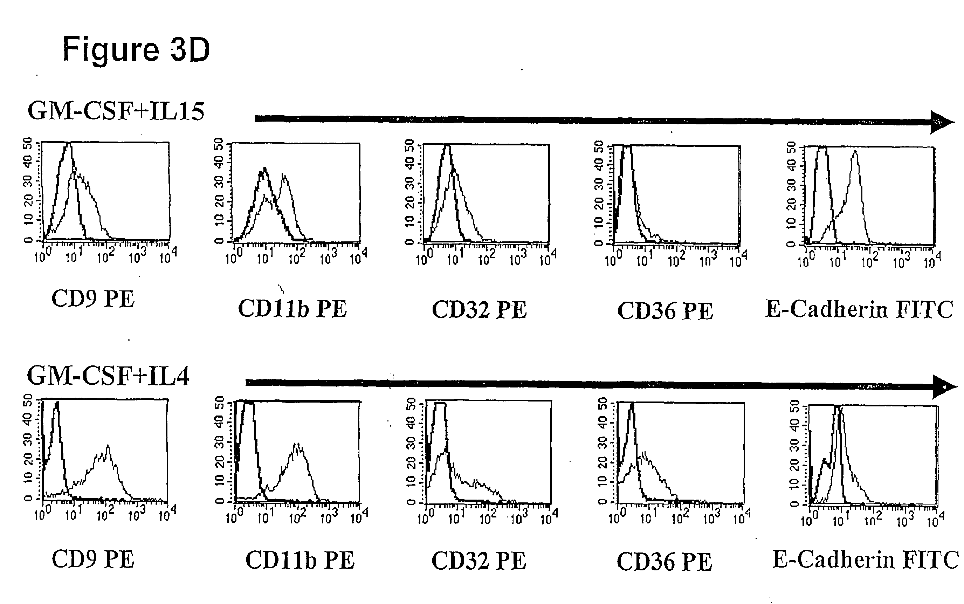

Fig. 3A depicts partial expression of Langerin (marker of Langerhans cells, a special type of dendritic cells) and CCR6 (chemokine receptor 6) in GM-CSF+IL-15-derived DCs. Fig. 3B depicts GM-CSF+IL-15-induced DCs expressing higher levels of Langerin when compared to GM-CFS+IL-4/TGFβ-induced DCs. Representative of three experiments, Fig. 3C depicts the fact that the generation of Langerhans cell-like DCs with GM-CSF and IL-15 is independent of TGFβ-1. Monocytes were cultured for 6 days with cytokines shown above the histograms with 300 ng/ml daily or without anti-TGFβ-1 antibody and stained as indicated. Fig. 3D depicts the fact that GM-CSF+IL-15-induced cells, but not GM-CSF+IL-4-induced cells, have Langerhans cells phenotype.

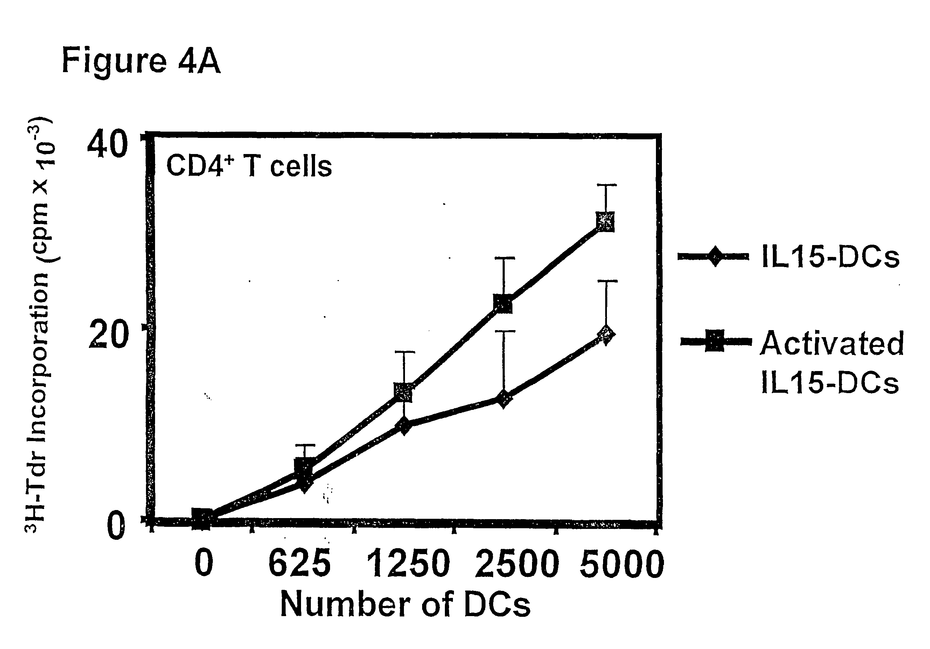

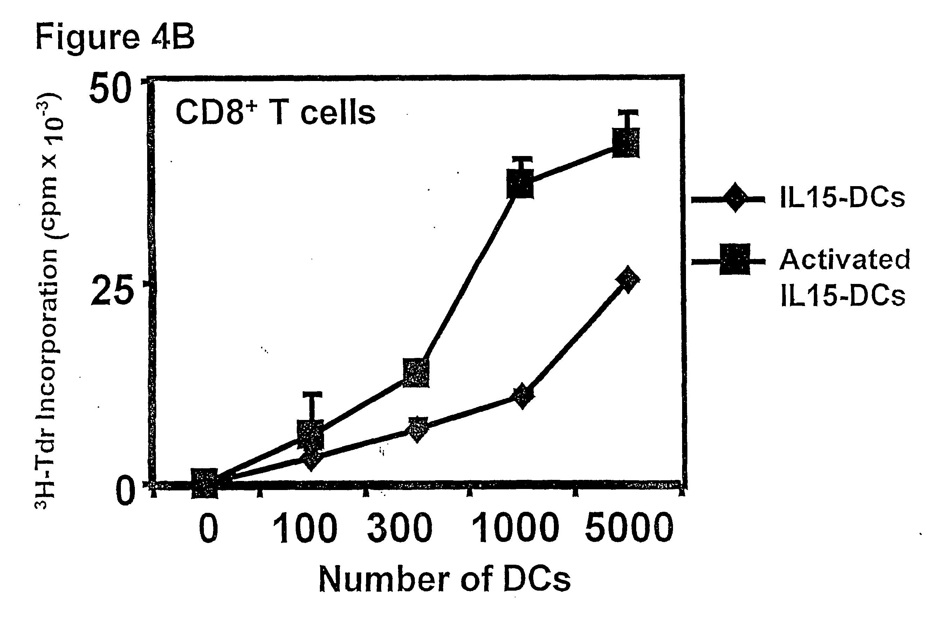

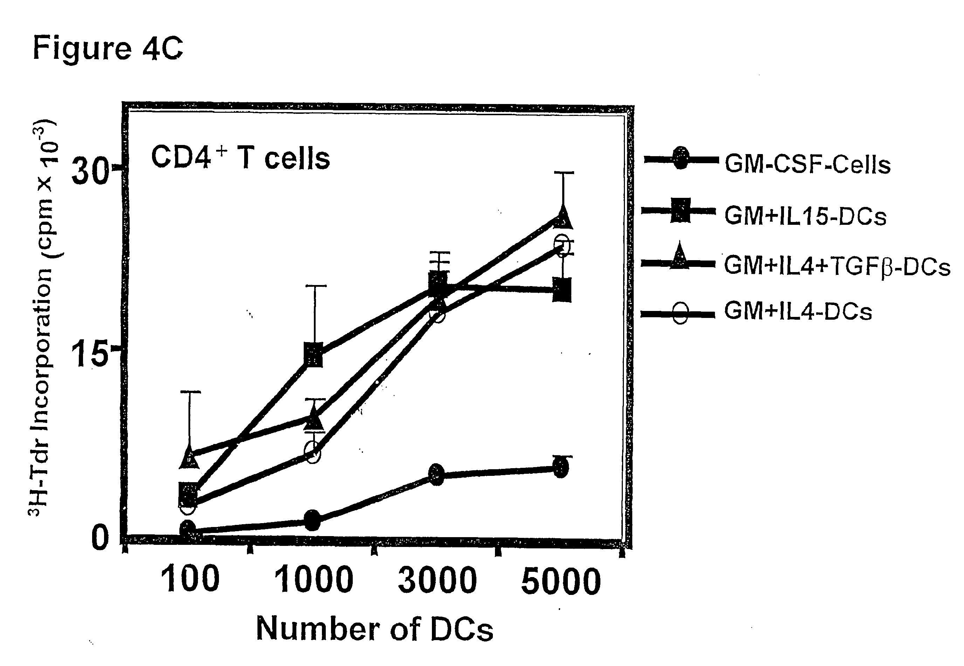

Fig. 4A, 4B, and 4C depict the allostimulatory capacity of GM-CSF+IL-15-derived DCs. DCs activated with poly (I):(C) or non-activated DCs were cultured as described herein and co-cultured with allogeneic CD4⁺ (Fig. 4A) or CD8⁺ T cells (1 x 10⁵) for 5 days (Fig. 4B). Cultures were pulsed with thymidine for 16 hours. Fig. 4C depicts a comparison of DCs cultured in GM-CSF+IL-15, GM-CSF+IL4, GM-CSF+IL-4+TGFβ-1, or GM-CSF alone in an allogeneic MLR.

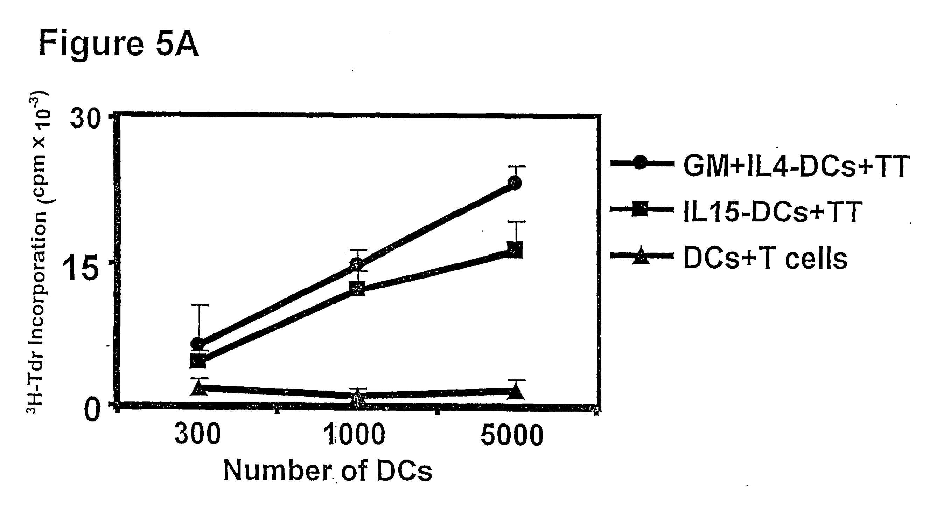

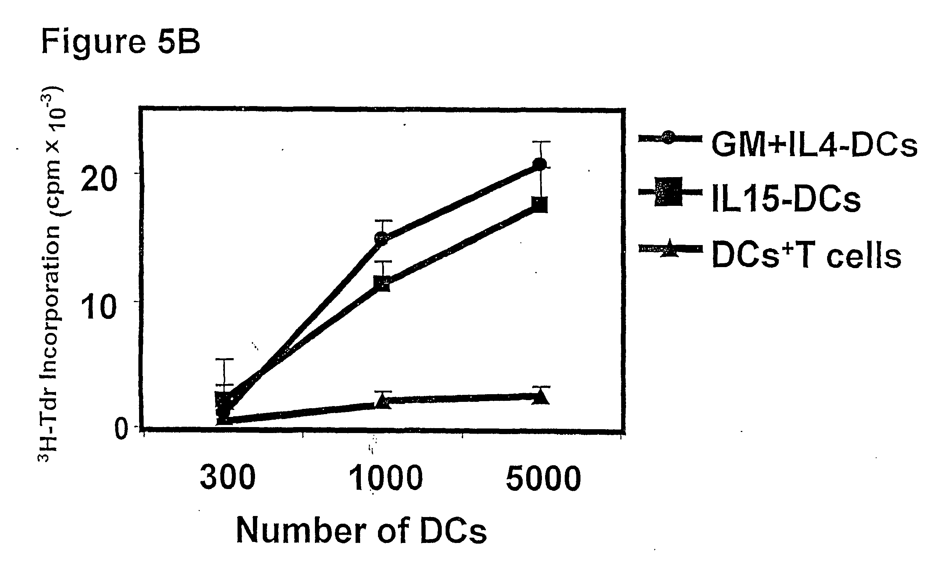

Fig. 5A and 5B depict presentation of TT (tetanus toxoid) or dying cell bodies by GM-CSF+IL-15-derived DCs. DCs were cultured as described herein. GM-CSF+IL-15-derived DCs and GM-CSF+IL-4-derived DCs were treated with TT (Fig. 5A) or dying cell bodies derived from Me 275 (Fig. 5B) at 37°C for 2 hrs. DCs were washed and cultured with 1 x 10⁵ naïve CD4⁺ T cells for 5 days. Cell cultures were pulsed with thymidine for the last 16 hours and incorporation of radionucleotide was measured by β-scintillation spectroscopy.

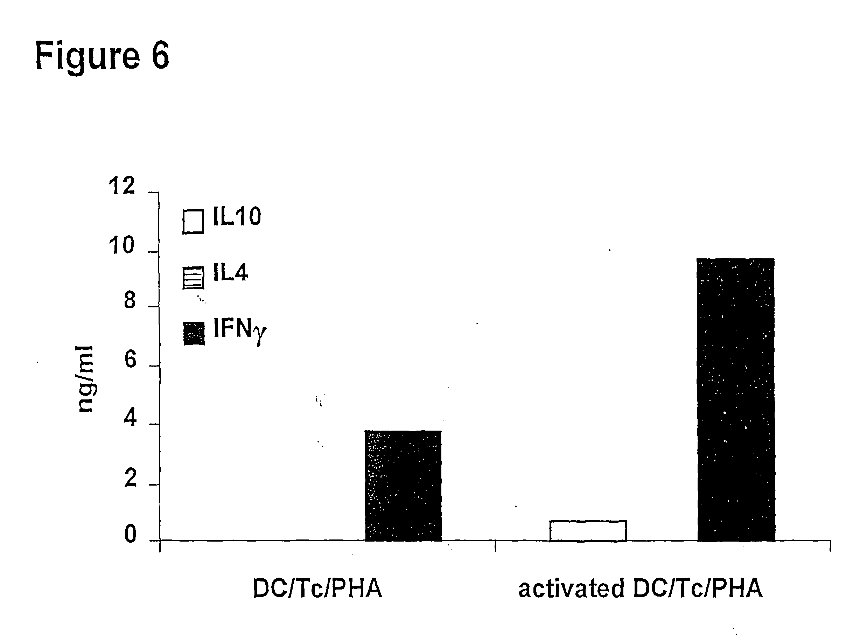

Fig. 6 depicts GM-CSF+IL-15 DCs inducing a distinct cytokine pattern in naive, allogeneic CD4+ T cells. Non-activated or activated GM-CSF+IL-15-derived DCs (5 x 10³) were co-cultured with CD4⁺ T cells (1 x 10⁵) for 5 days. The supernatants were collected, and the cells were stimulated with phytohaemagglutinin (PHA) for 24 hours. The supernatants were collected and assayed for cytokines using ELISA kits. Each point represents one independent experiment. The histograms represent the means of all experiments.

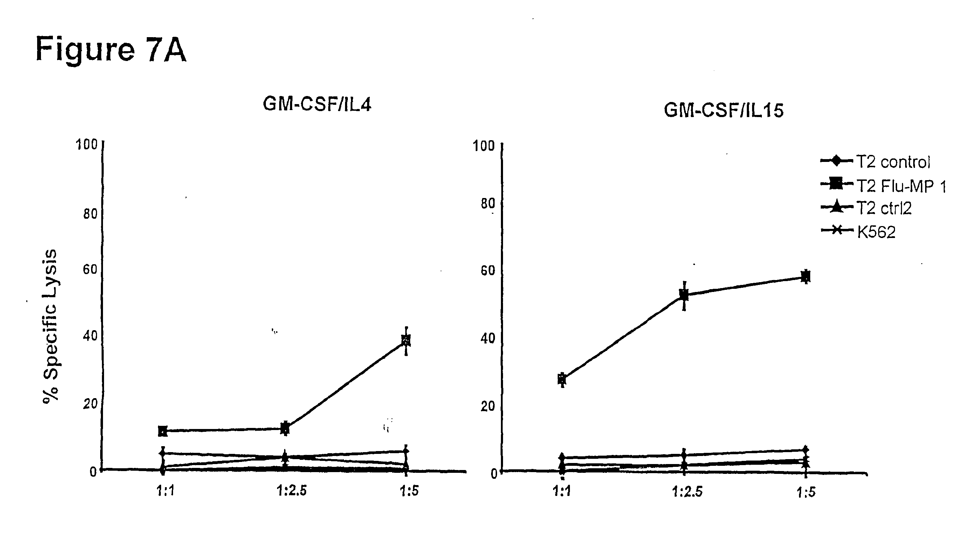

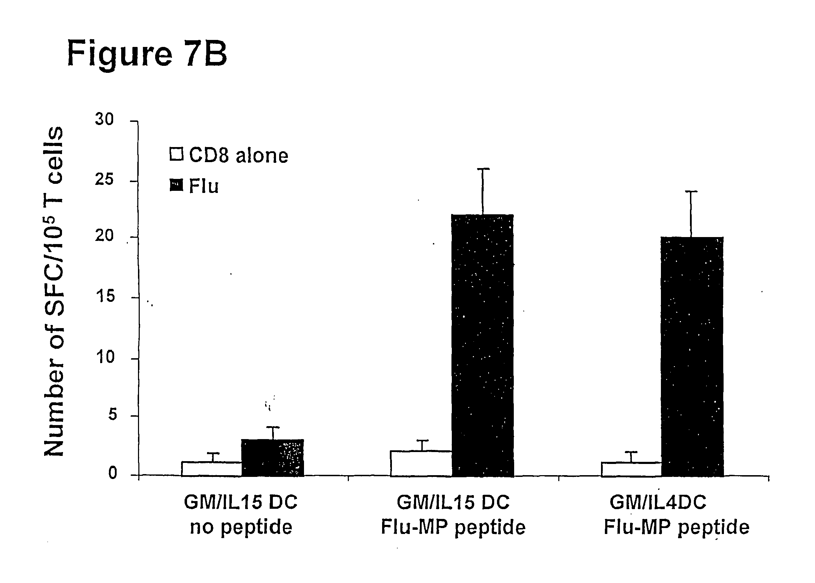

Fig. 7A and 7B depict the fact that GM-CSF+IL-15-derived DCs induce recall immune responses to viral antigen. HLA-A201+DCs were activated with a mix of macrophage cytokines (GM-CSF/IL-1/TNF-α/IL-6) or CD40L and pulsed for 16 hours with influenza-matrix peptide (Flu-MP). They were then harvested and used as stimulatory cells of autologous CD8⁺ T cells. Flu-MP antigen-specific effector CD8⁺ T cells were expanded for 14 days with restimulation with peptide-loaded DCs after the first week of culture. Effector cells were harvested and assayed for their ability to kill T2 cells (TAP-deficient HLA-A201+ hybridoma) unpulsed (negative control) or pulsed with Flu-MP peptide in a standard chromium release assay. Specific lysis was determined at 1:1, 1:2.5, 1:5, 1:1.75, and 1:30 E:T ratio (Fig. 7A). Effector cells were collected and assayed for number of IFN-γ-secreting cells per 100,000 effectors. Data represent the number of IFNγ⁺ cells responding to Flu-MP peptide-pulsed T2 cells (Fig. 7B).

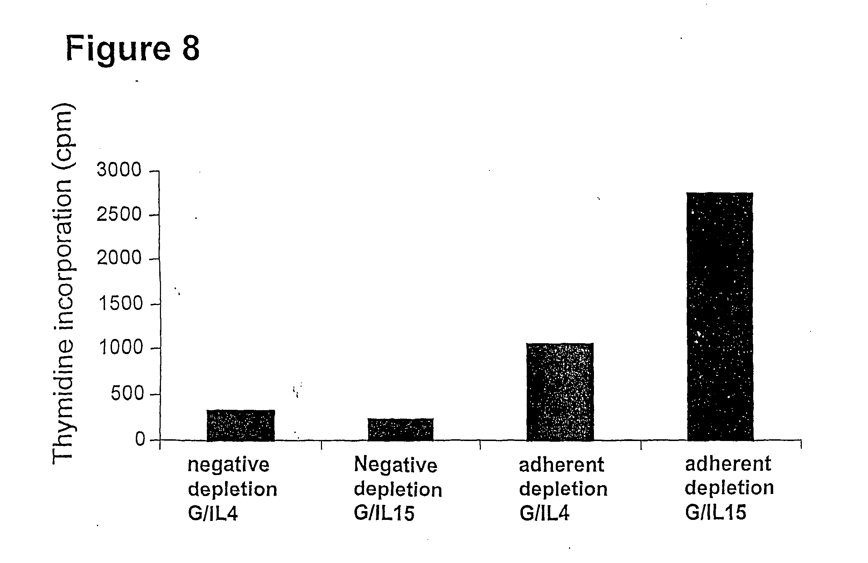

Fig. 8 depicts the lack of proliferation of cells obtained by culturing monocytes purified by the negative depletion protocol in the presence of IL-15 alone and IL-4 alone, and the prcliferation of cells obtained by culturing cells obtained by the adherent protocol in the presence of IL-15 alone and IL-4 alone. Cells obtained by both protocols were grown in the presence of IL-15 alone or IL-4 alone for five days. Afterwards, the cells were cultured in a 96 well plate for an additional 10 hours in the presence of ³H-thymidine. Thymidine uptake was then measured via beta counter.

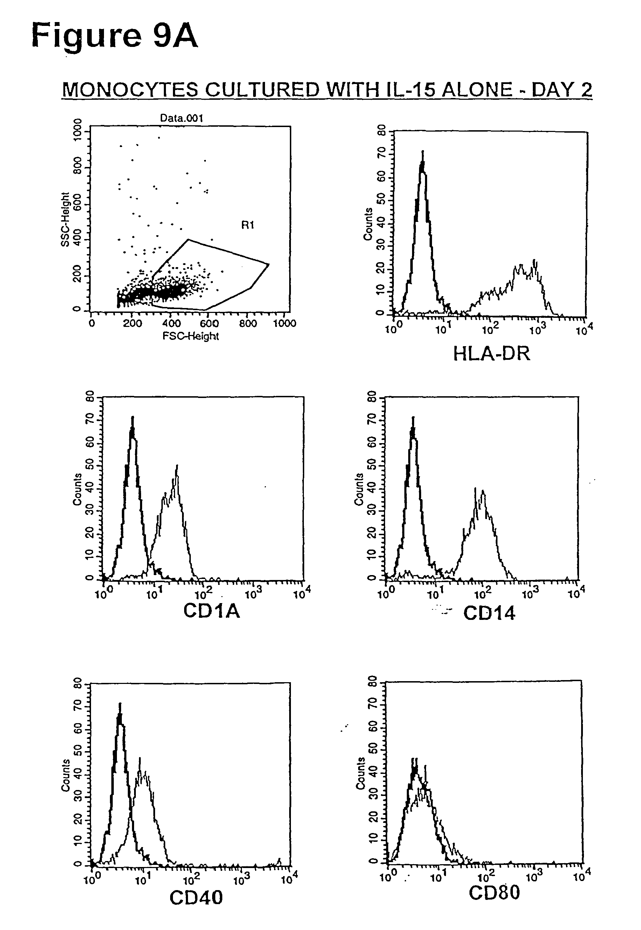

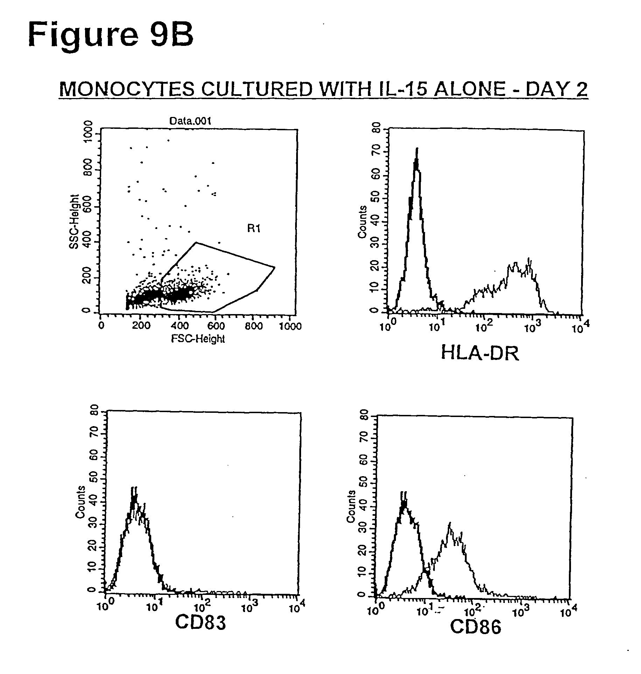

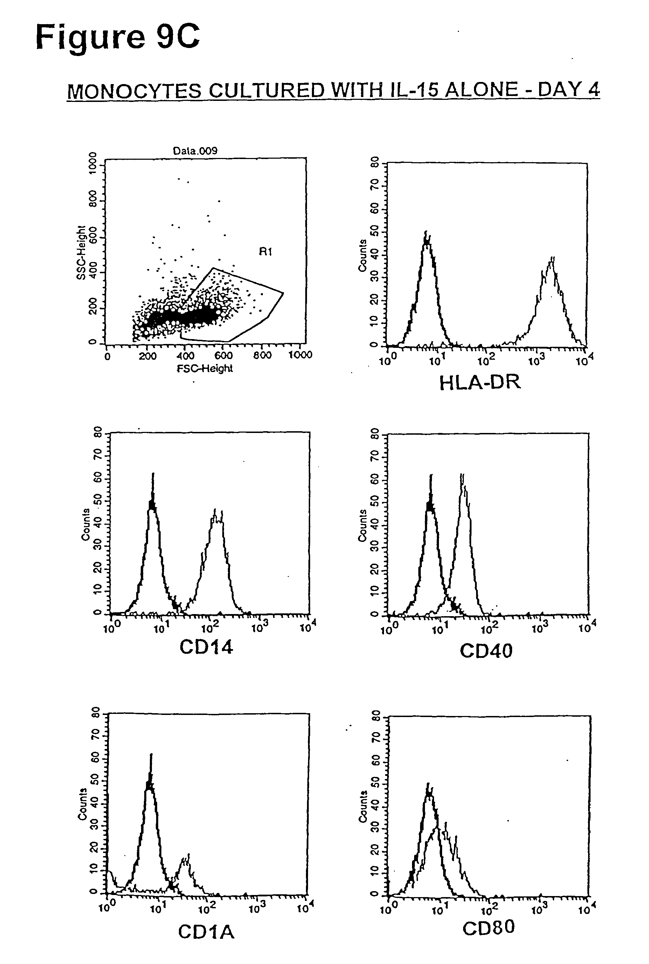

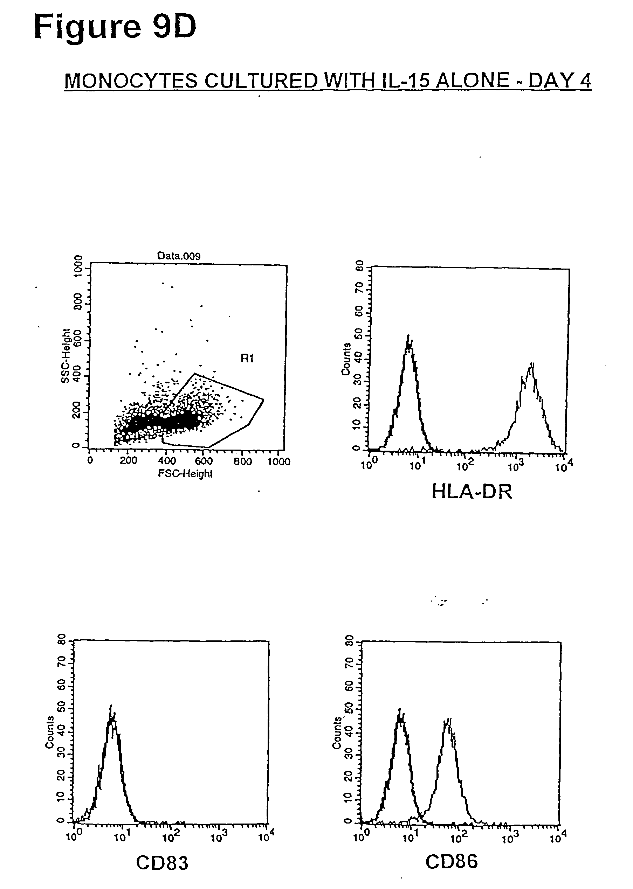

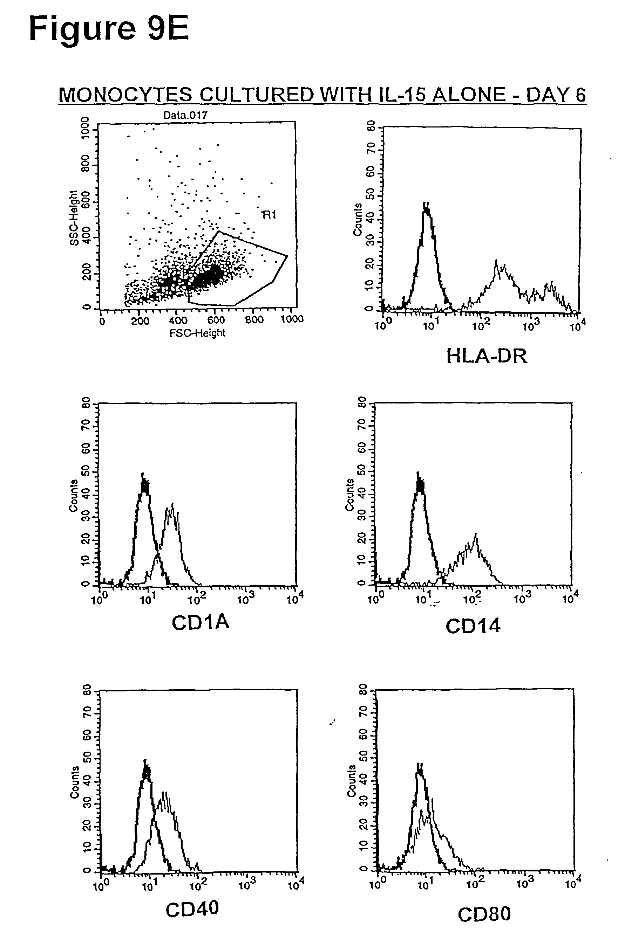

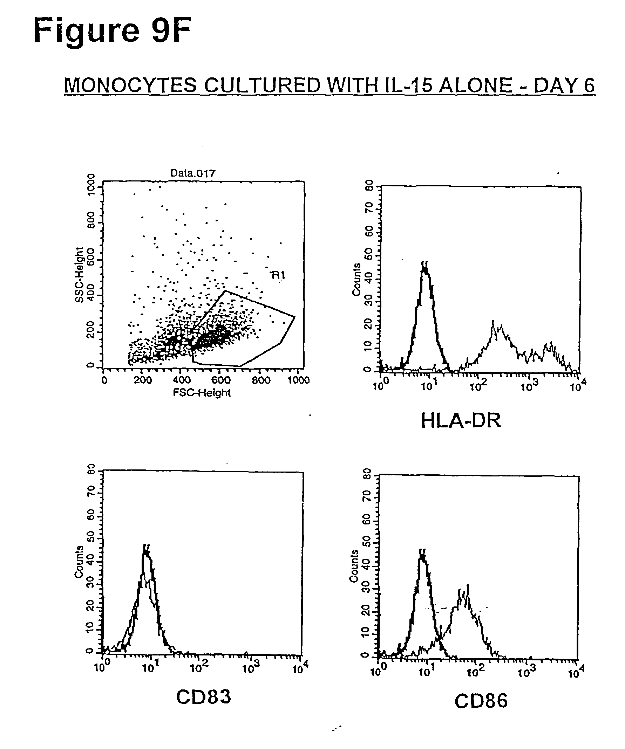

Fig. 9A, 9B, 9C, 9D, 9E and 9F depict time kinetic studies which indicate that the cells obtained by culturing purified monocytes in the presence of IL-15 are not immature dendritic cells. Monocytes purified by negative depletion were cultured in 6 well plates in the presence of IL-15. On Day 2 (Fig. 9A and 9B), Day 4 (Fig. 9C and 9D), and Day 6 (Fig. 9E and 9F), an aliquot of the cells was harvested and stained with anti-HLA-DR, anti-CD1a, anti-CD14, anti-CD40, anti-CD80, anti-CD83, and anti-CD86 and analyzed by flow cytometry.

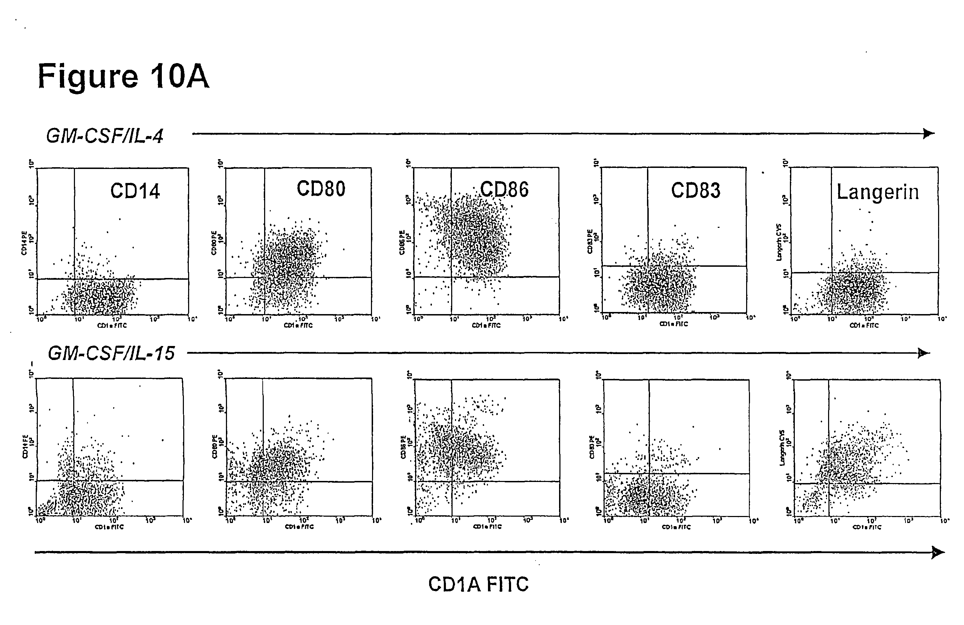

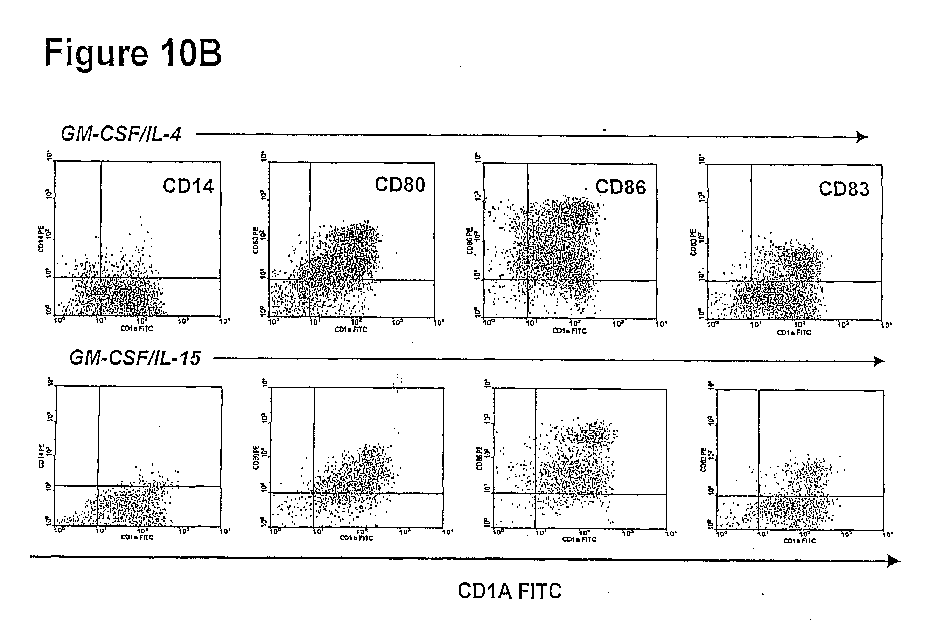

Fig 10A and 10B depict IL-15 in combination with GM-CSF can induce monocytes from blood of patients with stage IV melanoma to differentiate into dendritic cells under conditions approved for the clinical use of generated cells, i.e, in X-VIVO medium supplemented with 10% autologous serum. The induced dendritic cells can be further activated/induced to mature by a mix of macrophage cytokines (GM-CSF/IL-1/TNF-α/IL-6) all of which are approved for generation of cells to be injected to patients.

### DETAILED DESCRIPTION OF THE INVENTION

The present invention discloses compositions and methods of producing immature dendritic cells, activated dendritic cells, and mature dendritic cells, more preferably antigen-presenting dendritic cells, even more preferably of human origin, using interleukin-15 (IL-15). This invention resides, generally, in the use of IL-15 in combination with a growth factor, preferably GM-CSF, to cause or stimulate the differentiation or production of fully functional dendritic cells, immature dendritic cells, and/or activated dendritic cells from precursors thereof in vitro, ex vivo or in vivo. This invention also provides for the use of IL-15 alone to cause or stimulate the differentiation or production of precursor dendritic cells from monocytes in vitro, ex vivo or in vivo.

As demonstrated herein, the combination of IL-15 and GM-CSF is able to cause, induce, or stimulate the differentiation of monocytes and circulating DC precursors into dendritic cells ("DCs"). Enriched adherent blood CD14⁺ cells cultured for six days with GM-CSF and IL-15 differentiated into typical immature CD1a⁺HLA-DR⁺CD14⁻ DCs. As opposed to cultures made with GM-CSF+IL-4, 5-10% of GM-CSF+IL-15-derived DCs (IL-15 DCs) express Langerhans cell-specific markers (e.g., LAG-Langerin and CCR6). Langerhans cells (LCs) from the skin and other mucosal epithelia represent a subset of immature DCs that migrate to the draining lymph node after capture of antigens from invading pathogens. Such LCs subset may be removed from the composition by depletion, if desired.

The results presented herein further disclose that immature DCs obtained by using IL-15 in combination with GM-CSF efficiently capture soluble and particulate antigens as demonstrated using FTTC-Dextran and dying-cell bodies. Furthermore, agents such as LPS, TNF-α and CD40L diminish the IL-15 DCs' antigen-capturing capacity and promote their maturation (CD83⁺ DC-LAMP⁺). These DCs are also able to induce the proliferation of allogeneic CD4⁺ and CD8⁺ T cells and are able to process or present soluble (TT) and particulate (dying cell bodies) antigens to autologous T lymphocytes. Moreover, the mature DCs, loaded with Flu-MP peptide, are able to induce the expression ofINFy-producing CD8⁺ T cells and peptide-specific CTLs. CD4⁺ T cells cultured in the presence of these DCs produce high levels of IFNγ, low IL-10, and no IL-4, indicating Th₁-type skewing. Taken together, these results thus demonstrate a novel role for IL-15 in the generation of DCs, particularly LCs.

Interleukin-15 (IL-15) is a known cytokine, whose isolation, cloning and sequence were reported by Grabstein et al (U.S. Patent No. 5,747,024; and Grabstein et al. 1994. Science 264:965-968) and is presented in SEQ ID NO:1 (amino acid sequence given in SEQ ID NO:2). IL-15 uses the IL-2Rγ chain common to IL-2, IL-4, IL-7, IL-9 and IL-13, and various properties of IL-15 have been described in the art, such as a potent adjuvant effect when administered in combination with a vaccine antigen as disclosed in U.S. Patent No. 5,747,024. Within the context of the present invention, IL-15 refers to the polypeptide or corresponding nucleic acid as disclosed in SEQ ID NO:1, as well as any variant thereof, including any naturally-occurring derivatives thereof or homologs isolated from various mammalian species. Variants more generally encompass any polypeptide having one or several amino acid modifications as compared to SEQ ID NO:1, including mutation(s), deletion(s), insertion(s), substitution(s), alone or in various combinations(s). More particularly, variants as used herein are any polypeptide having the property to stimulate or induce production of dendritic cells from monocytes as disclosed herein, encoded by a nucleic acid sequence hybridizing, under conventional, moderate stringency, with the nucleic acid sequence disclosed in SEQ ID NO:1. Still preferably, IL-15 has at least 80% identity with the amino acid sequence disclosed in SEQ ID NO:2.

IL-15 is more preferably a recombinant IL-15, i.e., produced by expression, in any suitable host cell, of a nucleic acid molecule encoding such a polypeptide. Suitable host cells include, for example, mammalian cells, prokaryotic cells, or yeast cells. In this regard, recombinant human IL-15 is commercially available from various sources, including R&D Systems, Inc.(Minneapolis, MN, US). A preferred IL-15 for use in the present invention is a human IL-15, even more preferably a recombinant human IL-15. For use in the present invention, although essentially pure IL-15 preparations are preferred, it should be understood that any IL-15 preparation may be used, in combination with other factors, diluents, adjuvants, biological fluids, etc. Furthermore, while the process preferably utilizes an IL-15 polypeptide, the invention also includes the use of any IL-15-encoding nucleic acid molecule. In particular, the precursor cells may be contacted directly with a nucleic acid coding IL-15 incorporated in a suitable expression vector, or with a cell population containing an IL-15-encoding nucleic acid molecule and expressing IL-15. Such cells may further produce a growth factor such as GM-CSF. For in vivo production, the patient or subject may be administered with either IL-15 or any nucleic acid encoding IL-15 in a suitable expression vector. The concentration of IL-15 may be adjusted by the skilled artisan.

For the performance of the present invention, DC precursors are preferably contacted or treated with IL-15 and a growth factor, preferably GM-CSF, in vitro, ex vivo or in vivo. DC precursors include any cell or cell population which is capable of differentiating into dendritic cells in vitro, ex vivo or in vivo, upon treatment according to the present invention. Typical DC precursors include monocytes and circulating DC precursors. For in vitro or ex vivo uses, DC precursors may be obtained from various biological materials, including blood and bone marrow, preferably blood. Typically, DC precursors comprise peripheral blood mononuclear cells (PBMC) or peripheral blood monocytes isolated therefrom. PBMC can be prepared by conventional apheraesis, and monocytes isolated by Ficoll gradient, optionally coupled to depletion of T cells, B cells, and/or NK cells. Exemplary methods of isolating DC precursors useful in the present invention have been reported by Inaba et al (Inaba et al. 1992. J Exp Med 175:1157) and Romani et al (Romani et al. 1994. J Exp Med 180:83).

For in vitro and ex vivo applications, DC precursors may be contacted with IL-15, and optionally a growth factor such as GM-CSF, in any appropriate device, such as plate, well, flask, tube, etc., and preferably under sterile conditions. Typically, between 1 x 10⁴ to 1 x 10⁸ cells, more preferably between 1 x 10⁵ to 1 x 10⁷ cells are used, depending on the amount of DC precursors available or collected. The amount of cytokine and/or growth factor such as GM-CSF may be adjusted by the skilled person. Typically, IL-15 is used at a concentration of about 50 ng/ml to about 500 ng/ml, and more preferably about 100 ng/ml to about 300 ng/ml. GM-CSF may be employed at a concentration of about 10 ng/ml to about 300 ng/ml, and more preferably about 50 ng/ml to about 200 ng/ml. Similar concentrations or doses may be used for in vivo induction or stimulation of dendritic cell production or differentiation. The contacting cells may be contacted in vitro with the above reagents for about 24 hours to 15 days. Usually, immature DCs are produced and collected at Day 3-Day 7 after stimulation of DC precursors, preferably between 4-6 days post-treatment. Typically, 1 x 10⁶ DC precursor cells (e.g., monocytes) yield about 1 x 10³ immature DCs. Immature DCs obtained are particularly characterized by the expression of CD1a and HLA-DR, the absence of CD 14, and minimal levels or no expression of DC-LAMP (DC-lysosomal-associated membrane glycoprotein), CD40, CD80 and CD83.

Immature DCs may be collected and maintained in any suitable culture media, such as RPMI (GIBCO BRL, Grand Island, NY, US), X-VIVO 15 (Bio Whittaker, Inc., Walkersville, MD, US), AIMV (GIBCO BRL, Grand Island, NY, US), and DMEM (GIBCO BRL, Grand Island, NY, US), etc. The cells may be stored or frozen under conventional conditions. Quality, purity and lack of contamination may be assessed using conventional methods known in the art, such as antibody-labeling, dye labeling, etc.

A preferred aspect of this invention resides in a method for inducing or stimulating dendritic cell differentiation from precursors thereof, more particularly monocytes, comprising contacting the precursors with a combination of IL-15 and a growth factor, preferably GM-CSF, in vitro, ex vivo or in vivo. In a specific embodiment of this invention, immature dendritic cells or activated dendritic cells are produced from precursors of dendritic cells such as monocytes by contacting the precursors with a combination of IL-15 and a growth factor, preferably GM-CSF, in vitro, ex vivo or in vivo.

Immature DCs have utility in many applications, including diagnostic, therapy, vaccination, research, screening, gene delivery, etc. In particular, because of their potent immunogenic properties, DCs may be sensitized to an antigen and used to modulate an immune reaction or response in vivo, for instance to induce antigen-specific tolerance. As used herein, the modulation of the immune response by DCs includes increases, decreases, or changes to other types of immune responses.

Activated DCs have utility in many applications, including diagnostic, therapy, vaccination, research, screening, gene delivery, etc. In particular, because of their potent immunogenic properties, DCs may be sensitized to an antigen and used to cause, induce, or stimulate an immune reaction or response in vivo. They may also be used to produce, screen, or expand, in vitro or ex vivo, antigen-specific cytotoxic T lymphocytes (CTL).

In this respect, DCs may be sensitized to one or several antigens according to various techniques known in the art, such as by placing the DCs in contact with an antigen ("antigen pulsing"), an antigenic peptide ("peptide pulsing"), an antigenic protein complex, cells or cell membranes expressing antigens or antigenic peptides, texosomes, liposomes containing antigens or antigenic peptides, nucleic acids encoding antigens or antigenic peptides (possibly incorporated in plasmids or viral vectors), or total RNAs from a tumor cell. These methods have been disclosed, for instance, in International Application No. WO99/03499. Antigens may be tumor antigens, viral antigens, bacterial antigens, etc., more generally, any peptide or polypeptide against which an immune response or reaction is sought. The term "sensitized" indicates that the antigen or a portion thereof is exposed at the surface of the DCs, preferably in complex with molecules of the major histocompatibility complex (MHC).

Immature dendritic cells may also be used to produce membrane vesicles (also termed dexosomes), as described in WO99/03499, prior to or after antigen sensitization. In this regard, a particular object of this invention concerns a method of producing membrane vesicles, comprising (1) providing immature dendritic cells by contacting precursors thereof, more particularly monocytes, with IL-15 and GM-CSF, (2) culturing the dendritic cells under conditions allowing the release of membrane vesicles, and (3) isolating the membrane vesicles. Even more preferably, the method comprises, prior to Step (2), a sensitization of the DCs to one or several antigens. In Step (1), the DC precursors are preferably contacted with a combination of IL-15 and GM-CSF, as described herein. In Step (2), the DCs may be cultured in any conventional media, preferably in the presence of a stimulating compound and/or treatment and/or environment, to increase membrane vesicle release. Preferred stimulating conditions include culturing in the presence of cytokine(s), more preferably IL-10, gamma interferon (IFN-γ), or IL-12, irradiation and/or at low pH (e.g., pH=5), as described in WO99/03499. The membrane vesicles (or dexosomes) may be recovered, for instance, by centrifugation and/or chromatographic methods.

As indicated above, these membrane vesicles or dendritic cells can be used to modulate an immune response in a patient, in particular a mammal, more preferably a human. In this regard, the invention also relates to a method of modulating an immune response in a patient, comprising administering to the patient an effective amount of dendritic cells prepared in the presence of IL-15 or membrane vesicles derived therefrom. Even more preferably, the dendritic cells and/or membrane vesicles are sensitized to one or several antigens. In a specific embodiment, the DCs have been prepared from blood precursors, cultured in the presence of IL-15 and preferably a growth factor such as GM-CSF.

Another aspect of this invention lies in a method of modulating an immune response in a patient, via inducing dendritic cells in a patient, comprising the concurrent or sequential administration to the patient of an effective amount of IL-15 and a growth factor such as GM-CSF and/or maturation factor (e.g., CpG nucleotides or type I interferon). Sequential administration indicates that the compounds may be injected separately, in any order. Preferred administration routes include intravenous, intraarterial, intra-muscular, intra-dermal, and local (e.g., intra-tumoral or at the vicinity of a tumor site).

The DCs prepared according to the above methods may furthermore be activated or cultured in vitro or ex vivo under conditions allowing their maturation. In this respect, an object of the present invention resides in a method of producing mature DCs, comprising (1) preparing immature dendritic cells by contacting precursors thereof, more particularly monocytes, with IL-15 in the presence of a growth factor such as GM-CSF and (2) culturing the dendritic cells under conditions allowing their maturation, preferably in the presence of lipopolysaccharide (LPS), CD40L or poly(I):(C). Mature DCs show elevated expression of CD80, CD83 and CD86. Mature DCs may be sensitized to antigens, either prior, during, or after maturation.

Another object of the present invention resides in a method of producing antigen-specific CTL in vitro or ex vivo, comprising contacting antigen-sensitized dendritic cells as described above with a population of cells comprising T lymphocytes and recovering the activated CTLs which are specific for the antigen. The population comprising T lymphocytes may be isolated CD8+ T lymphocytes or peripheral blood mononuclear cells. Activated CTLs may be recovered and selected by measuring clonal proliferation, target cell lysis and/or cytokine release, as described in the examples provided herein. The antigen-specific CTLs may be further expanded by culture in the presence of antigen-sensitized DCs. This method is also applicable to the production of activated CD4+ T lymphocytes. Furthermore, the method may also be performed in vivo, to activate or increase a CTL response or a CD4+ response to an antigen in vivo.

The invention also includes compositions of matter comprising dendritic cells or membrane vesicles derived therefrom, obtainable as disclosed above. In particular, the invention relates to compositions comprising dendritic cells obtained by contacting in vitro or ex vivo precursors thereof in the presence of IL-15 and a growth factor such as GM-CSF, more particularly immature dendritic cells, even more particularly human immature dendritic cells. These dendritic cells may be sensitized to one or several antigens, as described above.

The invention also relates to compositions comprising membrane vesicles obtained from immature dendritic cells obtained by contacting in vitro or ex vivo precursors thereof in the presence of IL-15 and a growth factor such as GM-CSF. The invention further relates to compositions comprising IL-15 and GM-CSF, for concurrent or sequential use. The compositions may comprise any pharmaceutically acceptable carrier or excipient, e.g, buffer, saline solution and adjuvants. They can be used to induce or stimulate an immune response or reaction in vivo, e.g., for treating a patient with tumor or immune disease such as allergy, asthma, and/or autoimmune diseases. They are particularly suited for treating cancer in a patient

Another specific embodiment of this invention resides in a method of producing precursor dendritic cells from monocytes, comprising contacting the monocytes with IL-15 in vitro, ex vivo or in vivo. The invention also includes compositions of matter comprising precursor dendritic cells derived by contacting monocytes with IL-15 in vitro, ex vivo or in vivo.

### Monoclonal antibodies and recombinant growth factors and reagents

Monoclonal antibodies (mAbs) used in this study were: CD14, HLA-DR (Becton Dickinson, Franklin Lakes, NJ, US.); CD86, (PharMingen, San Diego, CA, US); CD40, HLA-AB, CCR6 (R&D Systems, Inc., Minneapolis, MN, US), CD1a (Dako Corp., Carpinteria, CA, US), CD80, and CD83 (Coulter/Immunotech, Fullerton, CA, US).

Recombinant human cytokines used in this study were: rhGM-CSF (100 ng/ml, Leukine, Immunex Corp., Seattle, WA, US); rbIL-4 (20 ng/ml, R&D Systems, Inc., Minneapolis, MN, US); rhIL-15 (200 ng/ml, R&D Systems, Inc., Minneapolis, MN, US); TGF-β1 (10 ng/ml, R&D Systems, Inc., Minneapolis, MN, US); and huCD40L (Immunex Corp., Seattle, WA, US).

Complete RPMI medium consisted of RPMI 1640, 1 % L-Glutamine, 1% penicillin/streptomycin, 50mM 2-ME, 1% sodium-pyruvate, 1% essential amino acids and heat inactivated 10% fetal calf serum (all from GIBCO BRL, Grand Island, NY, US). Poly(I):(C) was purchased from Pharmacia (Piscataway, NJ, U.S.A.), and lipopolysaccharide (LPS) was purchased from Sigma Chemical Co. (St Louis, MO, US).

### Cell culture and phenotypic analysis of monocytes derived dendritic cells

Peripheral blood monocytes were isolated by immunomagnetic depletion (Dynabeads; Dynal Biotech, Lake Success, NY, US) after separation of blood mononuclear cells followed by a Ficoll-Paque^{™} gradient The pellet was recovered for T cell purification, and monocytes were isolated negatively by positive magnetic depletion of T-cells, B-cells, and natural killer (NK) cells using purified anti-CD3, anti-CD16, anti-CD19, anti-CD56, and anti-glycophorin A antibodies. Afterwards, enriched CD14⁺ monocytes were cultured in 6 well plates (1 x 10⁶cells/well). These cells were cultured for 6 days in the presence of 100 ng/ml GM-CSF and 200 ng/ml IL-15 (GM-CSF+IL-15), or 100 ng/ml GM-CSF and 20 ng/ml IL-4 (GM-CSF+IL-4). On Day 6, cells were harvested and stained with anti-CD14-PE, anti-CD83-PE, anti-HLA-DR-PerCP, and anti-CD1a-FITC. In some experiments, DCs were activated with LPS (300 ng/ml), and their phenotypes were analyzed. The analysis was performed by FACS Calibur.

### Endocytotic activity of CD1a+ DC

The endocytotic activity of DCs was determined as follows. Activated with LPS (300 ng/ml) or non-activated GM-CSF+IL-15-derived DCs were harvested on Day 6 and incubated with 100 µg/ml FTTC-Dextran for 30 min at 37°C. As a control, part of the DCs was incubated with the same amount of FITC-Dextran on ice. The cells were washed with cold phosphate buffered saline (PBS) with 10% fetal calf serum and analyzed by FACS Calibur or confocal microscopy.

### Confocal Microscopy

Intracellular immunofluorescence staining was conducted as previously described (de Saint Vis, et al. 1998. Immunity 9:325-336; Valladeau et al. 2000. Immunity 12:71-81). On polylysin-coated coverslips, cells were fixed for 15 min with 4% paraformaldehyde in PBS. Afterwards, these cells were washed twice in 10 mM glycine in PBS and twice in PBS, and permeabilized with PBS containing 0.5% saponin and 1% bovine serum albumin (BSA) for 30 min. Coverslips were incubated for 30 min at room temperature with 5 µg/ml anti-DC-LAMP or anti-Langerin (gifts from Drs. Lebecque and Saeland, Schering-Plough, Dardilly, France). After three washes, cells were incubated for 30 min with secondary labeled donkey anti-mouse antibodies coupled to Texas red (Jackson Immuno Research Laboratories, Inc., West Grove, PA, US), washed, incubated with mouse pre-immune serum for 30 min, washed again, incubated for 30 min with a second primary antibody anti-HLA-DR directly coupled to fluorescein (Becton Dickinson. Franklin Lakes, NJ, US). Coverslips were mounted onto glass slides with Fluoromount (Southern Biotechnology Associates, Birmingham, AL, US). Confocal microscopy was performed using a TCS SP microscope equipped with argon and krypton ion lasers (Leica Microsystem, Heidelberg, Germany).

### T-cell proliferation

DCs cultured in GM-CSF+IL-15, GM-CSF+IL-4, or GM-CSF+IL-4/TGF-β1 were harvested, washed with complete RPMI and cultured with 1 x 10⁵ freshly isolated CD4⁺ or CD8⁺ allogeneic T cells derived from peripheral blood. In some experiments, DCs were activated with poly(I):(C) (6.5 µg/ml) for 16 hours. DC-T cell cultures were set up for 5 days in complete RPMI plus 10% human AB serum. Cells were pulsed for the last 16 hours with 0.5 mCi [³H]thymidine per well, and incorporation of the radionucleotide was measured by β-scintillation spectroscopy. To assay autologous T cell proliferation, GM-CSF+IL-15 or GM-CSF+IL-4 DCs were collected, washed, and then pulsed with tetanus toxoid (TT) for 48 hours. Afterwards, DCs were washed three times with PBS and then resuspended in complete RPMI plus 10% human AB serum. Cells were added in triplicate at various concentrations to 1 x 10⁵ autologous T cells/well in 96-well tissue culture plates (Falcon, Oxnard, CA, US). To assay capture of dying cell bodies by DCs, DCs were incubated for two hours with Me275 cell line bodies, which were prepared by treating with betulinic acid (10 µg/ml, Aldrich, Milwaukee, WI, US) for 48 hours. Afterwards, DCs were sorted based on forward/side scatter using a FACS Vantage. DCs were then co-cultured with autologous CD4⁺T cells. CD4⁺ or CD8⁺ T cells were isolated by the standard Ficoll-paque procedure followed by magnetic depletion of non-T cells (Dynabeads; Dynal Biotech, Lake Success, NY, US).

### Cytokine analysis

For cytokine analysis, supernatants were harvested 5 days after co-culture of GM-CSF+IL-15-derived DCs/CD4⁺ T cells, and the cells were re-stimulated with PHA (10 µg/ml) in fresh medium for 24 hours. Afterwards, the supernatants were collected and assayed for cytokine release by utilizing ELISA kits from R&D Systems (Minneapolis, MN, US).

### Generation of antigen-specific CTL

GM-CSF+IL-15-derived or GM-CSF+IL-4-derived DCs were stimulated with CD40L or with a mix of macrophage cytokines (GM-CSF/TNF-α/IL-1/IL-6). DCs were then washed and pulsed with 10 µg/ml of Flu-MP⁵⁸⁻⁶⁶ for further 18 hours. CD8⁺ effector T cells (1 x 10⁶) were isolated by magnetic beads separation and co-cultured with either non-pulsed DCs or with Flu-MP-pulsed DCs in 1 ml complete RPMI plus 10% human AB serum. All CTL cultures received IL-7 (10 ng/ml) at the establishment of culture and IL-2 (10 UI/ml) on Day 7 of culture. CTL were grown for 14 days prior to assay. CTL were used for cytotoxicity assay on Day 14. Effector cells (30 x 10³/well) were plated in 96-well round-bottom plates with T2 (1 x 10³/well) target cells incubated overnight with Flu-MP ⁵⁸⁻⁶⁶ and labeled with ⁵¹Cr (Amersham Pharmacia, Biotech, Inc., Piscataway, NJ, US). After 4 hours, supernatants were harvested using a harvesting frame and released chromium-labeled protein was measured using γ-counter (Packard Instruments Co, Meriden, CT, US). Percentage of antigen-specific lysis was then determined.

### INF-y Enzyme-linked Immunospot assay

To determine antigen-specific, IFN-γ-producing effector T cells, an enzyme-linked immunospot (ELISPOT) assay was used as described by Dhodapkar, et al. (Dhodapkar, et al. 1999. J Clin Invest 104:173-180). CD8⁺ T cells (1 x 10⁵/well) were added in triplicate to nitrocellulose-bottomed 96-well plates (MAHA S4510; Millipore Corp., Bedford, MA, US) precoated with the primary anti-IFN-γ mAb (1-D1K; Mabtech AB, Nacka, Sweden) in 50µl complete RPMI/well. For the detection of specific reactive T cells, autologous mature monocyte-derived DCs pulsed with MHC class I-restricted-peptides (Flu-MP) were added at 1 x 10⁴/well (final volume 100µl/well). After incubation for 20 hours, wells were washed 6 times, incubated with biotinylated secondary mAb against IFN-γ (7B6-1; Mabtech AB) for 2 hours, washed and stained with Vectastain Elite kit (Vector Laboratories, Inc., Burlingame, CA, US). Responses were counted as positive if a minimum of 10 spot forming cells (SFC)/2 x 10⁵ cells were detected and if the number of spots were at least twice that of the negative control wells.

### Comparison of the monocyte source in response to the presence of IL-15 alone

To determine if the monocyte source can affect the results obtained when the monocytes are treated with IL-15 alone, monocytes were obtained by the following two protocols: (1) negative depletion by magnetic beads and (2) an adherent protocol. In the negative depletion protocol, peripheral blood monocytes were isolated by immunomagnetic depletion (Dynabeads, Dynal Biotech, Lake Success, NY, US) after separation of blood mononuclear cells followed by a Ficoll-Paque^{™} gradient. The pellet was recovered for T cell purification and monocytes were isolated negatively by positive magnetic depletion of T cells, B cells, and NK cells using purified anti-CD3, anti-CD16, anti-CD 19, anti-CD56 and anti-glycophorin A antibodies. Afterwards, enriched CD14⁺ monocytes were cultured in 6 well plates (1 x 10⁶/well). An aliquot of these cells was cultured for 6 days in the presence of IL-15 alone and IL-4 alone, respectively. On Day 2, Day 4, and Day 6, cells were harvested, stained with anti-CD14, anti-CD83, anti-CD80, anti HLA-DR, anti-CD 1a, anti-CD40, and anti-CD86, and analyzed by FACS Calibur. In the adherent protocol, crude lymphocytes were cultured in 6-well plates for 2 hours, and the cells were washed three times with PBS containing 2% FSC to remove non-adherent cells. The adherent cells were then cultured with IL-15 alone and with IL-4 alone. Labeled thymidine was added to cells obtained by both the negative depletion protocol and the adherent protocol, and thymidine uptake was measured on Day 6 via β-counter.

Additional aspects and advantages of the present invention will be described in the following examples, which should be regarded as illustrative and not limiting the scope of the present application.

### Example 1: Monocytes cultured with GM-CSF+IL-15 yield immature DCs (IL-15 DCs)

A study was conducted to determine whether IL-15 may be involved in the differentiation of immature DC and most particularly LCs. Monocytes were highly purified by negative depletion using magnetic beads (98%) and cultured with IL-15 in combination with GM-CSF. After six days of culture with medium containing GM-CSF+IL-15, characteristic phase contrast morphology of IL-15 DCs indicated extensive cell aggregation. Similar cell aggregation was observed in cultures containing a combination of GM-CSF+IL-4. Representative phase contrast and Giemsa staining of the IL-15 DCs revealed extending veils, a morphology suggestive of DCs. Flow cytometry analysis showed that all the cells had acquired CD1a, lost CD14 and expressed relatively high levels of HLA-DR (Fig. 1A). Thus, monocytes cultured with GM-CSF+IL-15 differentiated into cells with a phenotype of immature DCs. Monocytes required 4-6 days of culture to fully differentiate into immature DCs (Fig. 1B) (hereinafter these cells are referred to as IL-15-DCs). After six days, 1 x 10⁶ monocytes yielded a mean 0.53 x 10⁶ immature DCs (mean of 5 experiments, SD=176.86). The generation of IL-15-derived DCs was independent of endogenous IL-4 production as determined by daily additions of neutralizing anti-IL4. Such treatment prevented the differentiation of monocytes into DCs in response to GM-CSF+IL-4, but not in response to GM-CSF+IL-15, while addition of anti-IL-15 or anti-IL-2Rγ chain blocked significantly the generation of CD1a⁺ DCs in response to GM-CSF+IL-15.

### Example 2: IL-15 DCs can be activated into mature DCs

As shown in Fig. 2A, IL-15-derived DCs express HLA-ABC Class I, low levels of CD86 and minimal levels CD40, CD80, CD83. Upon activation with LPS, cells show increased expression of CD40, CD80, CD86, and CD83. Confocal microscopy sections revealed the accumulation of intracellular DC-LAMP in the GM-CSF/IL-15 condition, a marker of mature DCs. The maturation of IL-15-derived DCs could be induced equally well with LPS and CD40L. At variance with DCs generated with IL-4, IL-15-derived DCs always display some activated cells as shown by spontaneous expression of DC-LAMP in 5-10% of the cells. As immature DCs, IL-15-derived DCs were capable of efficiently capturing antigens, e.g., FITC-Dextran (Fig. 2B). However, they lost this capacity upon maturation e.g., with LPS (Fig. 2A).

### Example 3: IL-15 DCs include LCs

CD34⁺ hematopoietic progenitor cells cultured with GM-CSF+TNF-α yield both interstitial DCs and LCs. Monocytes cultured with GM-CSF+IL-4 did not differentiate into LCs unless TGF-β1 was added to these cultures. To date, two markers distinguish LCs: CCR6, the receptor to MIP-3 α/β defensin, and LAG/Langerin, a lectin involved in the formation of Birbeck granules. As shown in Fig. 3A, up to 10% of IL-15-derived DCs expressed CCR6, while DCs generated with GM-CSF+IL-4 did not. Dual-immunofluorescence confocal microscopy of HLA-DR/Langerin was performed on monocyte-derived DCs cultured in vitro for 6 days with GM-CSF+IL-4, GM-CSF+IL-15, and GM-CSF+IL-4/TGF-β1. Confocal sections revealed the accumulation of intracellular Langerin in DCs generated by culturing monocytes with GM-CSF+IL-15 and GM-CSF+IL-4/TGF-β1 but not with GM-CSF+IL-4.

### Example 4: IL-15 DCs present soluble and particulate antigens to T cells

IL-15 DCs are efficient antigen-presenting cells which can induce the proliferation of allogeneic CD4⁺ and CD8⁺T cells (Fig. 4A and 4B). DCs generated by culturing of monocytes with either GM-CSF+IL-15, GM-CSF+IL-4, or GM-CSF+IL-4/TGF-β1 were comparable in their ability to induce allogeneic mixed lymphocyte reaction (MLR) (Fig. 4C). They were considerably more efficient than cells generated with GM-CSF alone (Fig. 4C). IL-15 DCs activated for 24 hours with poly (I):(C) (Fig. 4A, B), CD40L or LPS showed increased allo-stimulatory capacity. The capacity to present antigens to autologous CD4⁺ T cells was also assayed. Thus, IL-15 DCs loaded with soluble tetanus toxoid (TT) induced autologous CD4⁺ T cells to proliferate (Fig. 5A). Furthermore, IL-15 DCs that had captured dying cell bodies also induced the proliferation of autologous T lymphocytes (Fig. 5B).

### Example 5: IL-15 DCs induce CD4⁺ T cells to produce INF-γ

The capacity of IL-15 DCs to skew T cell differentiation was assessed by co-culturing them with naïve CD4⁺CD45RA⁺ T cells for five days. Afterwards, cells were washed and stimulated with PHA, and supernatants were assayed for cytokine production. Non-activated IL-15 DCs induced T cells to produce high amounts of IFN-γ, but not IL-4 or IL-10 (Fig. 6). Co-cultures of T cells and stimulated IL-15 DCs with Poly (I):(C) contained higher levels of IFN-γ (Fig. 6). Then, IL-10 could be observed in supernatants (Fig. 6).

### Example 6: Cytotoxicity assay

The ability of IL-15 DCs to stimulate MHC class-I restricted CTL responses was evaluated. IL-15 DCs were pulsed with Flu-MP peptide and used to stimulate purified CD8⁺ T cells. After two seven-day culture cycles performed in the presence of IL-7 (both cycles) and IL-2 (second cycle), the elicited T cells were able to efficiently kill T2 cells loaded with Flu-MP peptide (55% specific lysis at a 2.5:1 ratio and 60% specific lysis at a 5:1 E:T ratio). T cell lines, which were generated by co-culture with unloaded IL-15 DCs (control DCs) induced less than 5% specific lysis (Fig. 7A). However, as shown in Fig. 7B, IL-15 DCs loaded with Flu-MP peptide were comparable to GM-CSF+IL-4 DCs in stimulating the generation of peptide-specific CTL precursors able to produce IFN-γ. Unpulsed DCs did not induce specific IFN-γ producing cells (Fig. 7B).

### Example 7: Culture of monocytes in the presence of IL-15 alone

When purified monocytes obtained by the negative depletion protocol were grown in the presence of IL-15 and GM-CSF, the resulting GM-CSF+IL-15 DCs were CD1a⁺ and CD14⁻, indicative of immature dendritic cells. However, when these monocytes were cultured with IL-15 alone, the resulting cells were CD1a⁺ and CD 14⁺, indicative of precursor dendritic cells and not immature dendritic cells. As shown in Fig. 8, the purified monocytes obtained by the negative depletion protocol did not proliferate when cultured in the presence of IL-15 or IL-4. Time kinetic studies presented in Fig. 9A-9F further demonstrate that the cells obtained after culturing these purified monocytes in IL-15 alone were not immature dendritic cells.

As shown in thymidine uptake studies illustrated in Fig. 8, when adherent cells obtained by the adherent protocol were cultured in the presence ofIL-15 alone, the resulting cell culture showed evidence of proliferation. These results indicated that the adherent cells not only included monocytes but also contaminating T cells, B cells, and NK cells, known to respond to IL-15 by proliferation.

As indicated above, IL15 in conjunction with GM-CSF induces highly purified monocytes to differentiate into immature DCs. The cells generated under these conditions are in many ways comparable to DCs generated with GM-CSF+IL4: 1) the same phenotype; 2) the same capacity to capture FITC-Dextran and dying cell bodies; 3) the same capacity to mature in response to various stimuli; 4) the same ability to process and present soluble and particulate antigens; 5) the same ability to induce the differentiation of T cells into the Th1 pathway; and 6) the same ability to induce the differentiation of CD8 T cells into specific CTL. Two differences were observed between DCs generated in the presence of IL-4 and DCs generated in the presence of IL-15: 1) IL-15 cultures included some mature DCs as shown by expression of DC-LAMP; and 2) IL-15 cultures contained Langerhans cells. This latter observation is relevant to IL-15, which is a product of KCs, the cells that fully surround the LCs within the epidermis. Thus, monocytes that extravasate from dermal vessels would differentiate into interstitial DCs when encountering the GM-CSF and IL-4 produced by dermal mast cells; but would differentiate into Langerhans cells when encountering the GM-CSF and IL-15 produced by KCs.

### Example 8: IL-15 induces monocytes from patients with metastatic cancer to differentiate into dendritic cells

As shown in Fig. 10, the adherent fraction of peripheral blood mononuclear cells give rise, upon culturing with IL-15 and GM-CSF to cells with the phenotype of dendritic cells including a Langerhans cell component as determined by surface expression of Langerin. These cells are obtained in culture performed under conditions approved for clinical use and the reinjection of cultured cells into patient. The cells are generated in X-VIVO culture medium supplemented with 2%-10% autologous serum.

### References

Kupper et al. ,1988. "Keratinocyte derived T-cell growth factor (KTGF) is identical to granulocyte macrophage colony stimulating factor (GM-CSF)," J Invest Dermatol 91:185-188.
Grabstein et al. 1994. "Cloning of a T cell growth factor that interacts with the beta chain of the interleukin-2 receptor," Science 264:965-968.
Burton et al. 1994. "A lymphokine, provisionally designated interleukin T and produced by a human adult T-cell leukemia line, stimulates T-cell proliferation and the induction of lymphokine-activated killer cells," Proc Natl Acad Sci USA 91:4935-4939.
Bulfone-Paus et al. 1997. "Interleukin-15 protects from lethal apoptosis in vivo," Nat Med 3:1124-1128.
Tagaya et al. 1996. "IL-15: a pleiotropic cytokine with diverse receptor/signaling pathways whose expression is controlled at multiple levels," Immunity 4:329-336.
Kennedy et al. 2000. "Reversible defects in natural killer and memory CD8 T cell lineages in interleukin 15-deficient mice," J Exp Med 191:771-780.
Ku et al. 2000. "Control of homeostasis of CD8+ memory T cells by opposing cytokine," Science 288:675-678.
Mohamadzadeh et al. 1996. "Interleukin-15 expression by human endothelial cells: up-regulation by ultraviolet B and psoralen plus ultraviolet A treatment," Photodermatol Photoimmunol Photomed 12:17-21.
Mohamadzadeh et al.1995. "Ultraviolet B radiation up-regulates the expression of IL-15 in human skin," J Immunol 155:4492-4496.
Oppenheimer-Marks et al. 1998. "Interleukin 15 is produced by endothelial cells and increases the transendothelial migration of T cells in vitro and in the SCID mouse-human rheumatoid arthritis model in vivo," J Clin Invest 101:1261-1272.
Caux et al. 1992. "GM-CSF and TNF-alpha cooperate in the generation of dendritic Langerhans cells," Nature 360:258:261.
Valladeau et al. 2000. "Langerin, a novel C-type lectin specific to Langerhans cells, is an endocytic receptor that induces the formation of Birbeck granules," Immunity 12:71-81.
Ma et al. 2000. "The pleiotropic functions of interleukin 15: not so interleukin 2-like after all," J Exp Med 191:753-755.
Dhodapkar et al. 1999. "Rapid generation of broad T-cell immunity in humans after a single injection of mature dendritic cells," J Clin Invest 104:173-180.

## Claims

1. Use of immature or mature dendritic cells or membrane vesicles obtained by contacting dendritic cell precursors with a composition comprising interleukin-15 and at least one growth factor for the preparation of a pharmaceutical composition for modulating an immune response in a patient.

2. Use of claim 1, wherein the dendritic cells are mature dendritic cells which have been contacted with a maturation agent.

3. Use of any of claims 1 or 2, wherein the precursors are contacted with the composition comprising interleukin-15 and at least one growth factor between 24 hours and 15 days.

4. Use of any of claims 1 to 3, wherein the precursors are monocytes.

5. A method for producing immature, mature or activated dendritic cells or isolated membrane vesicles from dendritic cell precursors in vitro or ex vivo, comprising contacting said precursors with a composition comprising interleukin-15 and at least one growth factor.

6. Immature, mature or activated dendritic cells or isolated membrane vesicles obtainable according to the method of claim 5.

7. A pharmaceutical composition comprising the immature, mature or activated dendritic cells or isolated membrane vesicles according to claim 6.

8. Use of interleukin-15 and at least one growth factor for the preparation of a pharmaceutical composition for producing immature or mature dendritic cells in a patient, wherein the interleukin-15 is formulated for concurrent or sequential administration with the at least one growth factor.

9. Use of interleukin-15 for the preparation of a dendritic cell precursor expressing CD1a+CD14+CD86+ by contacting monocytes in vitro or ex vivo.

10. A method for producing antigen-specific cytotoxic T lymphocytes in vitro or ex vivo comprising providing antigen sensitized dendritic cells obtained by contacting monocytes with interleukin-15 and at least one growth factor and subsequent sensitizing of said dendritic cells to said antigen, and contacting said antigen-sensitized dendritic cells with a population of cells comprising T lymphocytes to form activated antigen-specific cytotoxic T lymphocytes.

11. Use of antigen-specific cytotoxic T lymphocytes obtained according to the method of claim 6 for the preparation of a pharmaceutical composition for increasing an immune response in a patient.


SEQUENCE LISTING

<110> Banchereau, Jacques
      Mohamadzadeh, Mansour
      Palucka, Anna

<120> Compositions and Methods For Producing Antigen-Presenting Cells

<130> 10347/21605

<150> US 60/203,571
<151> 2000-05-11

<150> PCT/US01/15300
<151> 2001-05-11

<160> 2

<170> PatentIn version 3.0

<210> 1
<211> 1202
<212> DNA
<213> Homo sapiens

<220>
<221> CDS
<222> (317)..(805)

<400> 1
   tgtccggcgc cccccgggag ggaactgggt ggccgcaccc tcccggctgc ggtggctgtc      60
   gccccccacc ctgcagccag gactcgatgg agaatccatt ccaatatatg gccatgtggc     120
   tctttggagc aatgttccat catgttccat gctgctgctg acgtcacatg gagcacagaa     180
   atcaatgtta gcagatagcc agcccataca agatcgtatt gtattgtagg aggcatcgtg     240
   gatggatggc tgctggaaac cccttgccat agccagctct tcttcaatac ttaaggattt     300
   accgtggctt tgagta atg aga att tcg aaa cca cat ttg aga agt att tcc 352
   Met Arg Ile Ser Lys Pro His Leu Arg Ser Ile Ser
   1 5 10
   atc cag tgc tac ttg tgt tta ctt cta aac agt cat ttt cta act gaa 400
   Ile Gln Cys Tyr Leu Cys Leu Leu Leu Asn Ser His Phe Leu Thr Glu
   15 20 25
   gct ggc att cat gtc ttc att ttg ggc tgt ttc agt gca ggg ctt cct 448
   Ala Gly Ile His Val Phe Ile Leu Gly Cys Phe Ser Ala Gly Leu Pro
   30 35 40
   aaa aca gaa gcc aac tgg gtg aat gta ata agt gat ttg aaa aaa att 496
   Lys Thr Glu Ala Asn Trp Val Asn Val Ile Ser Asp Leu Lys Lys Ile
   45 50 55 60
   gaa gat ctt att caa tct atg cat att gat gct act tta tat acg gaa 544
   Glu Asp Leu Ile Gln Ser Met His Ile Asp Ala Thr Leu Tyr Thr Glu
   65 70 75
   agt gat gtt cac ccc agt tgc aaa gta aca gca atg aag tgc ttt ctc 592
   Ser Asp Val His Pro Ser Cys Lys Val Thr Ala Met Lys Cys Phe Leu
   80 85 90
   ttg gag tta caa gtt att tca ctt gag tcc gga gat gca agt att cat 640
   Leu Glu Leu Gln Val Ile Ser Leu Glu Ser Gly Asp Ala Ser Ile His
   95 100 105
   gat aca gta gaa aat ctg atc atc cta gca aac aac agt ttg tct tct 688
   Asp Thr Val Glu Asn Leu Ile Ile Leu Ala Asn Asn Ser Leu Ser Ser
   110 115 120
   aat ggg aat gta aca gaa tct gga tgc aaa gaa tgt gag gaa ctg gag 736
   Asn Gly Asn Val Thr Glu Ser Gly Cys Lys Glu Cys Glu Glu Leu Glu
   125 130 135 140
   gaa aaa aat att aaa gaa ttt ttg cag agt ttt gta cat att gtc caa 784
   Glu Lys Asn Ile Lys Glu Phe Leu Gln Ser Phe Val His Ile Val Gln
   145 150 155
   atg ttc atc aac act tct tga ttgcaattga ttctttttaa agtgtttctg     835
   Met Phe Ile Asn Thr Ser
               160
   ttattaacaa acatcactct gctgcttaga cataacaaaa cactcggcat ttaaaatgtg 895
   ctgtcaaaac aagtttttct gtcaagaaga tgatcagacc ttggatcaga tgaactctta 955
   gaaatgaagg cagaaaaatg tcattgagta atatagtgac tatgaacttc tctcagactt 1015
   actttactca tttttttaat ttattattga aattgtacat atttgtggaa taatgtaaaa 1075
   tgttgaataa aaatatgtac aagtgttgtt ttttaagttg cactgatatt ttacctctta 1135
   ttgcaaaata gcatttgttt aagggtgata gtcaaattat gtattggtgg ggctgggtac 1195
   caatgct 1202

<210> 2

<211> 162

<212> PRT

<213> Homo sapiens

<400>  2

Met Arg Ile Ser Lys Pro His Leu Arg Ser Ile Ser Ile Gln Cys Tyr
1               5                   10                  15

Leu Cys Leu Leu Leu Asn Ser His Phe Leu Thr Glu Ala Gly Ile His
            20                  25                  30

Val Phe Ile Leu Gly Cys Phe Ser Ala Gly Leu Pro Lys Thr Glu Ala
        35                  40                  45

Asn Trp Val Asn Val Ile Ser Asp Leu Lys Lys Ile Glu Asp Leu Ile
    50                  55                  60

Gln Ser Met His Ile Asp Ala Thr Leu Tyr Thr Glu Ser Asp Val His
65                  70                  75                  80

Pro Ser Cys Lys Val Thr Ala Met Lys Cys Phe Leu Leu Glu Leu Gln
                85                  90                  95

Val Ile Ser Leu Glu Ser Gly Asp Ala Ser Ile His Asp Thr Val Glu
            100                 105                 110

Asn Leu Ile Ile Leu Ala Asn Asn Ser Leu Ser Ser Asn Gly Asn Val
        115                 120                 125

Thr Glu Ser Gly Cys Lys Glu Cys Glu Glu Leu Glu Glu Lys Asn Ile
    130                 135                 140

Lys Glu Phe Leu Gln Ser Phe Val His Ile Val Gln Met Phe Ile Asn
145                 150                 155                 160

Thr Ser